# EUROPEAN PATENT SPECIFICATION

(11) **EP 2 233 062 B1**
(45) Date of publication and mention of the grant of the patent: **21.08.2013**
(21) Application number: 08870182.6
(22) Date of filing: 14.11.2008
(51) Int. Cl.: A61B 1/00, A61B 5/07, H04N 5/225, G06T 7/20, G06F 17/30, G06F 19/00

(54) **SCENE CHANGE DETECTION DEVICE AND SCENE CHANGE DETECTION PROGRAM**
VORRICHTUNG UND PROGRAMM ZUR ERKENNUNG VON SZENENÄNDERUNGEN
DISPOSITIF DE DÉTECTION DE CHANGEMENT DE SCÈNE ET PROGRAMME DE DÉTECTION DE CHANGEMENT DE SCÈNE

(30) Priority: 09.01.2008 JP 2008002183
(43) Date of publication of application: 29.09.2010
(73) Proprietor: Olympus Corporation, Shibuya-ku Tokyo 151-0072 (JP)
(72) Inventor: MATSUZAKI, Hiroshi, Tokyo 151-0072 (JP)
(74) Representative: von Hellfeld, Axel
(86) International application number: PCT/JP2008/070784
(87) International publication number: WO 2009/087816

(56) References cited:
- EP-A1- 1 862 106
- WO-A1-2006/100808
- JP-A- 2006 041 794
- JP-A- 2006 217 045
- JP-A- 2006 334 297
- JP-A- 2007 075 157

## Description

### TECHNICAL FIELD

The present invention relates to a scene-change detection device and a scene-change detection program for detecting a scene change image at the position where a scene is changed in a sequence of continuously-taken images or a sequence of frame images of a moving image.

### BACKGROUND ART

A moving image is composed of a sequence of an enormous number of continuous images, and to create a summary image sequence by detecting useful images from the sequence of continuous images is a useful technical field. Much the same is true of a sequence of continuously-taken still images. For example, an in-vivo image taken with a capsule endoscope is taken about every 0.5 second from when the capsule endoscope is swallowed through the mouth until the capsule endoscope is carried out of the body, and a sequence of about 60000 continuous images is obtained. These images are images of the digestive tract taken sequentially, and displayed on a workstation or the like and observed to give a diagnosis. However, it takes more than an hour to sequentially observe all the images, i.e., as many as about 60000 images, so it is hoped a technique for conducting an observation efficiently will be proposed.

Conventionally, various methods for detecting an image at the position where a scene is changed (a scene change image) from a sequence of continuous images like a moving image have been proposed. It is conceivable that such a scene change image is used to efficiently conduct an observation of large quantities of images. As a method for detecting a scene change image, for example, there is generally well known a method of comparing an amount of change in feature between adjacent images (frames) with a predetermined threshold and detecting the image as a scene change image if the amount of change in feature exceeds the threshold.
For example, document JP2006217045A discloses an index image generation method and apparatus which has a scene-change frame extracting section, the frame can be divided into several areas as base for scene detection. Uses weight coefficients and characteristics of the regions. The disclosed method and apparatus is based on a feature-value region set by the user. The distribution of characteristics is based on a detected motion.
Document EP1862106A1 discloses a capsule endoscope image display controller which calculates similarity between image and temporally continuous image captured by endoscope moving in digestive organ, and calculates movement amount of feature area shown in each image. The controller calculates similarity between an image and a temporally continuous image included in image series captured by capsule endoscope moving in digestive organ, and calculates movement amount of feature area shown in each image.

Furthermore, there is an example of a proposal enabling to make a change to a generated scene change image sequence by providing an input means for changing a threshold of an inter-frame change and setting a desired threshold selected from a plurality of thresholds (for example, see Patent Document 1).

Patent Document 1: Japanese Laid-open Patent Publication No. 2006-41794

### DISCLOSURE OF INVENTION

### PROBLEM TO BE SOLVED BY THE INVENTION

However, in the technology disclosed in Patent Document 1, although a threshold value can be selectively set, a user manages to obtain a desired summary image sequence through repetition of threshold change setting by trial and error. Still, in a process using a predetermined threshold, it is not easy to obtain an appropriate scene change image sequence capturing a feature of an image that the user wants to detect.

Especially, in a case of an image of inside the body cavity taken by the above-mentioned capsule endoscope, the interest of a user, such as a doctor, in observation often does not coincide with the magnitude of a change of a whole image. The user may want to detect, as a scene change image, an image of a site even though the whole image shows a gradual change; conversely, an image of a site may not have to be detected as a scene change image even though the whole image shows a rapid change. In conventional technologies, it is difficult to meet such demands sufficiently.

The present invention has been made in consideration of the above, and an object of the present invention is to provide a scene-change detection device and a scene-change detection program capable of detecting a scene change image in accordance with a feature of an image from a sequence of continuous images.

### MEANS FOR SOLVING PROBLEM

To solve the problems as described above and achieve the object, a scene-change detection device according to the present invention is a scene-change detection device as described in claim 1.

A scene-change detection program according to the present invention causes a computer to execute the steps described in claim 7.

### EFFECT OF THE INVENTION

According to a scene-change detection device and a scene-change detection program of the present invention, detection of an image-to-image change can be made on a condition of image-to-image change detection in consideration of a feature amount of a feature region. Consequently, it is possible to detect a scene change image in accordance with a feature of an image to which a user pays attention from a sequence of continuous images.

### BRIEF DESCRIPTION OF DRAWINGS

FIG. 1 is a functional block diagram illustrating a configuration of a scene-change detection device according to a first embodiment of the present invention.
FIG. 2 is a schematic flowchart illustrating a procedure of a scene-change detection process according to the first embodiment.
FIG. 3 is a schematic flowchart illustrating a more detailed process example of processes at Steps S105 and S106 in FIG. 2.
FIG. 4 is a schematic explanatory diagram illustrating how, for example, three images in a continuous image sequence are extracted in time-series order.
FIG. 5 is a schematic diagram illustrating a difference in change among five continuous images A to E between when a region feature is not considered as in conventional technologies and when the region feature is considered as in the first embodiment.
FIG. 6 is a schematic diagram illustrating an example of an image sequence from which a scene change image is detected.
FIG. 7 is a schematic flowchart illustrating a processing example according to a second embodiment of the present invention.
FIG. 8 is a configuration diagram schematically illustrating a capsule endoscope system including a scene-change detection device according to an example as a workstation.

### EXPLANATIONS OF LETTERS OR NUMERALS

22 Feature extracting unit
23 Detecting unit

### BEST MODE(S) FOR CARRYING OUT THE INVENTION

A scene-change detection device and a scene-change detection program as best modes for carrying out the present invention are explained below with reference to the accompanying drawings. The present invention is not limited to embodiments described below, and various variants can be made without departing from the scope of the present invention.

### First embodiment

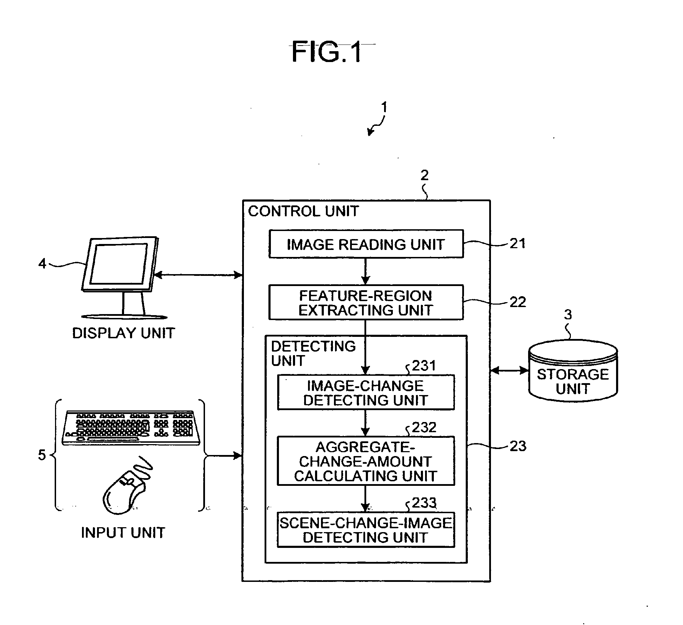

FIG. 1 is a functional block diagram illustrating a configuration of a scene-change detection device according to a first embodiment of the present invention. A scene-change detection device 1 shown in FIG. 1 is realized by a computer including hardware, such as a CPU, a ROM, and a RAM, and includes a control unit 2, a storage unit 3, a display unit 4, and an input unit 5.

The control unit 2 schematically includes a calculating function and a control function. The storage unit 3 stores therein image information on a sequence of time-series continuous images like a moving image, and is composed of a database and the like. The display unit 4 is composed of an LCD, an ELD, a CRT, or the like, and displays various information including images of a scene change image sequence (a summary image sequence), a processing result, on a display screen thereof. The input unit 5 is composed of a keyboard, a mouse, a pointer, and the like, and an input operation of various information or an instruct operation for processing an image is performed through the input unit 5.

The control unit 2 includes an image reading unit 21, a feature-region extracting unit 22, and a detecting unit 23. The image reading unit 21 reads images in the continuous image sequence stored in the storage unit 3.
The feature-region extracting unit 22 extracts at least one feature region from each of the images sequentially read by the image reading unit 21 by using the existing feature-region extraction technology, which results in dividing a whole area of a target image into a plurality of regions. The extraction of a feature region in the present embodiment is a concept including recognition of the feature region. The recognition of the feature region can be made in such a way that the whole area of the target image is divided into a plurality of regions, and a process of setting a level of importance with respect to each of the regions on the basis of a feature of each region or the like is performed to associate with a level of importance. The feature region means, for example, a portion of an image showing a feature of the image or a desired object on the image on an image-to-image basis, but sometimes a whole image is a feature region.

The detecting unit 23 sets a condition of image-to-image change detection on the basis of a feature amount of a feature region extracted by the feature-region extracting unit 22 and calculates an image-to-image change amount, thereby detecting a change among a plurality of images. The detecting unit 23 includes an image-change detecting unit 231, an aggregate-change-amount calculating unit 232, and a scene-change-image detecting unit 233.

The image-change detecting unit 231 calculates an image-to-image change amount of each region in a whole area of a target image (including an extracted feature region, a region other than the feature region, and a common region and a mismatched region between the target image and an image to be compared that are generated in accordance with movement of the feature region with respect to the image to be compared) with respect to the image to be compared. The aggregate-change-amount calculating unit 232 sets a condition of image-to-image change detection to be varied on the basis of a feature amount of the extracted feature region, and calculates an image-to-image change amount revised on a region-by-region basis in accordance with the condition of image-to-image change detection, and then accumulates a result of the calculation thereby calculating a statistic as an image-to-image change amount of the whole area of the image. Here, the aggregate-change-amount calculating unit 232 sets a condition of image-to-image change detection so that an image-to-image change of each region in a whole area of a target image is weighted on the basis of a feature amount of a feature region extracted by the feature-region extracting unit 22. Consequently, a region-by-region image change amount is varied relative to a threshold of determination of an image change on the basis of a feature amount of a feature region. The scene-change-image detecting unit 233 compares the statistic on the image-to-image change amount calculated by the aggregate-change-amount calculating unit 232 with a predetermined threshold of determination of an image change, thereby detecting an image having a statistic exceeding the threshold as a scene change image (a summary image). The scene-change-image detecting unit 233 generates a scene change image sequence using detected scene change images (summary images), and serves for the time-series display on the display screen of the display unit 4.

The CPU included in the computer, the scene-change detection device 1 having the above units in the control unit 2 thereof, executes a calculation process for a scene-change detection process by reading a scene-change detection program for executing the scene-change detection process according to the present first embodiment from the ROM in the computer and loading the scene-change detection program in the RAM. The scene-change detection program according to the present first embodiment can be recorded on a computer-readable recording medium, such as a flexible disk, a CD-ROM, a DVD-ROM, or a flash memory, so that the scene-change detection program can be widely distributed. Therefore, the scene-change detection device according to the present first embodiment can be configured to include an auxiliary storage device that can read any of the above-mentioned various recoding media.

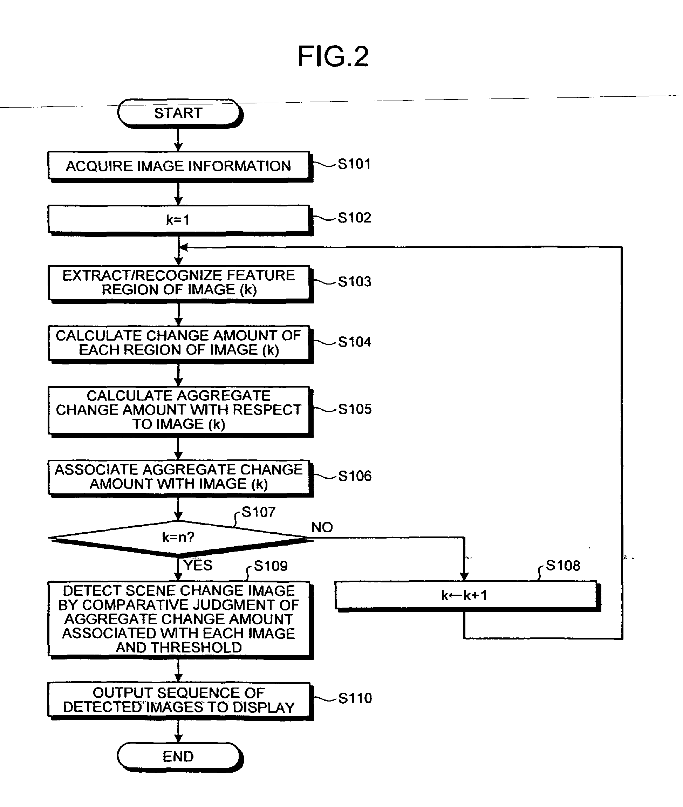

FIG. 2 is a schematic flowchart illustrating a procedure of the scene-change detection process according to the present first embodiment. First, the image reading unit 21 acquires information on n, the number of all images composing a continuous image sequence, the image size, and the like from the storage unit 3, and sequentially reads the images (Step S101). Then, the image reading unit 21 sets a variable k, which indicates what number image for identifying an image to be processed, at 1, which indicates the first image (Step S102). Then, the feature-region extracting unit 22 extracts a feature region from an image (k) with the k-th image (k) as an image to be processed (Step S103). By this process, it turns out that a whole area of the image (k) has a region part other than the feature region, and the whole area of the image (k) is divided into at least a plurality of regions. Namely, the region part other than the extracted feature region can also be treated as a feature region when a condition of detection is set, and it is possible to apply the way of thinking that a whole area of an image is divided into regions and each region is treated as a feature region.

Then, the image-change detecting unit 231 calculates an image-to-image change amount of each region in the whole area of the target image (k) (including the extracted feature region, a region other than the feature region, and a common region and a mismatched region between the target image (k) and an image to be compared that are generated in accordance with movement of the feature region with respect to the image to be compared to be described later) with respect to the image to be compared (Step S104). Then, the aggregate-change-amount calculating unit 232 sets a condition of image-to-image change detection on the basis of a feature amount of the extracted feature region, and calculates an image-to-image change amount revised on a region-by-region basis in accordance with the condition of image-to-image change detection, and then accumulates a result of the calculation thereby calculating a statistic (an aggregate change amount) as an image-to-image change amount of the whole area of the image (Step S105). The calculated statistic is associated with the image (k) as an image change amount of the image (k) (Step S106).

Such processes are repeated in the same manner by incrementing the variable k by +1 until the variable k reaches n, the number of all images (Steps S107 and S108). When the calculation of the statistic with respect to all the images is completed, the scene-change-image detecting unit 233 detects a scene change image by comparative judgment of the statistic associated with each image and a predetermined threshold (Step S109), and outputs a scene change image sequence composed of the detected scene change images to the display unit 4 (Step S110).

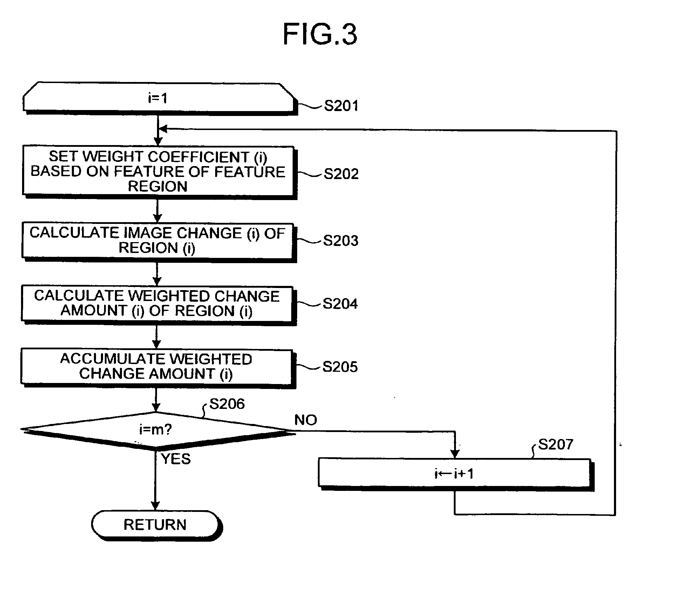

A more detailed process example of the processes at Steps S105 and S106 in FIG. 2 is explained with reference to FIG. 3. Here, the number of regions into which each image is divided on the basis of extraction of a feature region is denoted by m, and a variable identifying each region is denoted by i. First, the variable i is set at 1 (Step S201). Then, the aggregate-change-amount calculating unit 232 sets a weight coefficient (i) of a region (i) on the basis of a feature amount of an extracted feature region of a corresponding image (Step S202). Namely, with respect to the region (i), a condition of image-to-image change detection is set so that an image-to-image change of the region (i) is weighted on the basis of the feature amount of the feature region. Then, the image-change detecting unit 231 calculates an image change amount (i) of the region (i) with respect to an image to be compared (Step S203). Furthermore, the aggregate-change-amount calculating unit 232 calculates a weighted image change amount (i) of the region (i) by multiplying the calculated image change amount (i) by the weight coefficient (i) (Step S204), and accumulates the calculated weighted image change amount (i) to aggregate the weighted image change amount (Step S205). Such processes are repeated in the same manner by incrementing the variable i by +1 until the variable i reaches m, the number of all the regions (Steps S206 and S207).

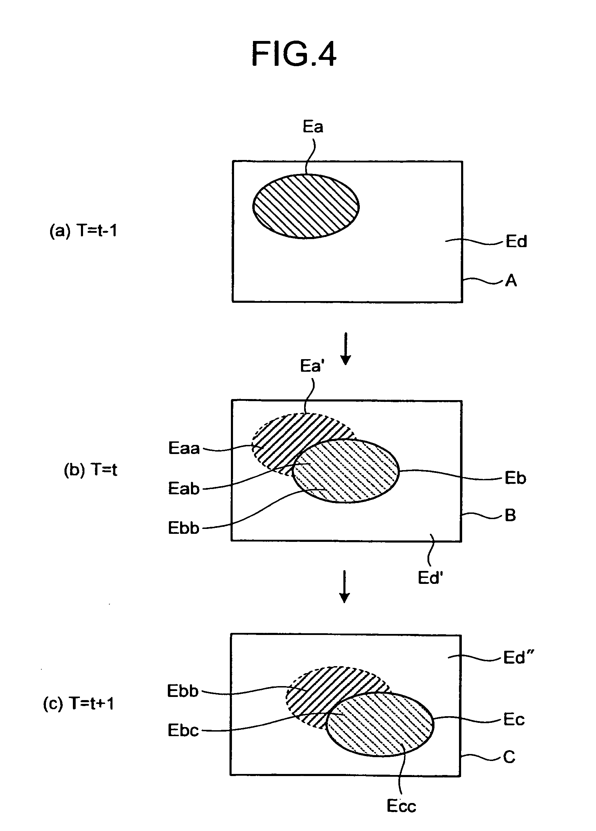

Subsequently, a process for weighting region by region in a whole area of a target image according to the present first embodiment is explained with reference to FIG. 4. FIG. 4 is a schematic explanatory diagram illustrating how, for example, three images in a continuous image sequence are extracted in time-series order. Images A, B, and C are images taken at timings T = t - 1, T = t, and T = t + 1 in time-series order, respectively, and the image B shall be an attention image to be processed. Furthermore, respective identical feature regions of the images A, B, and C each extracted by the feature-region extracting unit 22 and having a feature amount equivalent to a high level of importance shall be denoted by Ea, Eb, and Ec, respectively.

First, as for the image A, in accordance with the extraction of the feature region Ea, a whole area of the image A is divided into the feature region Ea and a region Ed other than the feature region Ea. Here, it can be thought that the region Ed is also one feature region. Next, the feature region Eb extracted in the attention image B to be processed is the one that the feature region Ea on the image A to be compared moves to another position, and the original position of the feature region Ea is shown as a feature region Ea' on the image B. In this manner, in accordance with the movement of the attention region with respect to the image A to be compared, a common region Eab and mismatched regions Eaa and Ebb between the feature region on the image B and the feature region on the image A to be compared are generated. Furthermore, in accordance with the movement of the feature region, the region Ed also changes to a region Ed' that is not included in both the feature regions Ea and Eb. In the present embodiment, for example, as for the image B, the regions Eaa, Eab, Ebb, and Ed' are treated as regions in a whole area of the image B.

When there is such a movement of the feature region, from a feature amount of each region, it is considered that the common region Eab has the highest level of importance. Thus, weight on an image-to-image change amount of the common region Eab is set high. And, it is considered that the mismatched region Eaa, which is the one that the common region Eab is excluded from the feature region Ea', and the mismatched region Ebb, which is the one that the common region Eab is excluded from the feature region Eb, each have a level of importance lower than that of the common region Eab. Consequently, with respect to the mismatched regions Eaa and Ebb, a corresponding weight coefficient is reduced, and then an image-to-image change amount is calculated. Furthermore, it is considered that the region Ed', which is not included in the feature regions before and after the movement, has a level of importance lower than those of the mismatched regions Eaa and Ebb. Consequently, with respect to the region Ed', a weight coefficient is reduced to be lower than those of the mismatched regions Eaa and Ebb, and then an image-to-image change amount is calculated. Namely, a condition of image-to-image change detection is set so that an image-to-image change of each of the regions Eab, Eaa, Ebb, and Ed' with respect to the image A is weighted by multiplying the image-to-image change by a different weight coefficient depending on a level of importance of each of the regions Eab, Eaa, Ebb, and Ed'.

Then, the weighted image-to-image change amount of each of the regions Eab, Eaa, Ebb, and Ed' is accumulated to calculate a statistic, whereby an aggregate image-to-image change amount of the whole image can be calculated. Namely, a value of an overall image-to-image change amount taking respective levels of importance of the regions in the whole area of the image B into consideration is taken as an image-to-image change amount of the image B. The image-to-image change amount calculated in this way is an image-to-image change amount revised on the basis of the feature amount of the extracted feature region.

Much the same is true on a process when the next image C in time-series order is an object to be processed.

In FIG. 3, if a feature region which is originally low in level of importance is to be extracted, the relation of level of importance described above can be set in an opposite manner. In this case, for example, the common region is treated as a region of the lowest level of importance.

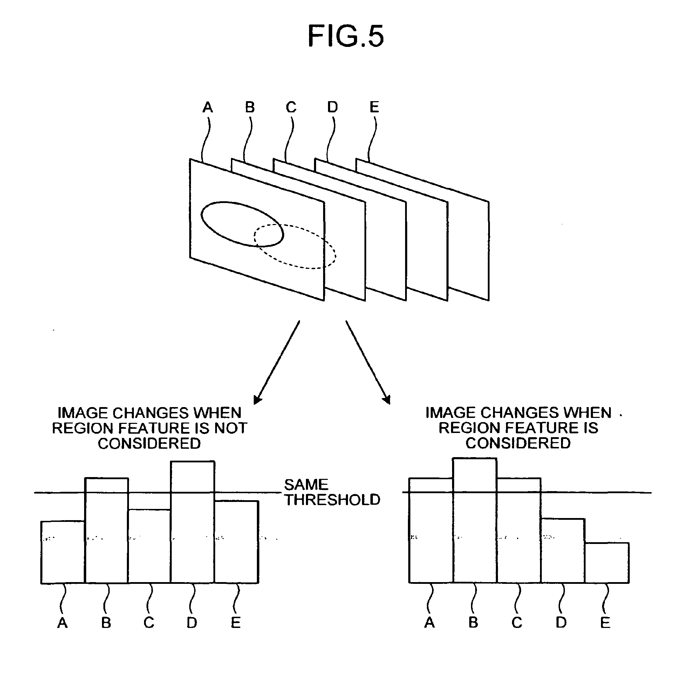

In this manner, when an aggregate image-to-image change amount is obtained as a statistic, the aspect of change differs from a case of an image-to-image change amount obtained by a simple comparison of whole image. For example, FIG. 5 is a schematic diagram illustrating a difference in image-to-image change among five continuous images A to E between when a region feature is not considered as in conventional technologies and when the region feature is considered as in the present first embodiment. When the region feature is not considered as in conventional technologies, a simple comparison of whole image is performed, and if a simple image-to-image change amount of each image exceeds a predetermined threshold (for example, the images B and D), the image is detected as a scene change image. On the other hand, in the present first embodiment, in a case of even the same images A to E, a feature region characterizing image content is extracted from an image to be processed, and a whole area of the image to be processed is divided into a plurality of regions including the feature region, and then a condition of image-to-image change detection is set with each region in the whole area of the image to be processed including a common region and a mismatched region that are generated in accordance with movement of the feature region with respect to the image to be compared attached with a level of importance depending on a feature amount of the feature region by a weight coefficient, whereby a region-by-region image-to-image change amount is varied relative to a predetermined threshold depending on image content. Thus, for example, as for the images A to C, an image-to-image change amount is relatively varied on the side to increase larger than that is in the simple comparison, and even though the predetermined threshold is the same, the images A to C are detected as a scene change image exceeding the threshold. On the other hand, for example, as for the images D and E, an image-to-image change amount is relatively varied on the side to decrease smaller than that is in the simple comparison, and even though the predetermined threshold is the same, the image-to-image change amount does not exceed the threshold, and the images D and E are not detected as a scene change image.
Therefore, in detection of a scene change image using the same threshold, a detected scene change image differs between the conventional method and the case of the present first embodiment; however, in the case of the present first embodiment, determination is made by reflecting a level of importance of a feature region in the form of weighting,
and thus it is possible to detect an appropriate scene change image based on a feature of a target image.

Namely, in the present first embodiment, when an image has content that one wants to extract the image as a scene change image as much as possible, even if an actual image-to-image change amount is small, the image-to-image change amount is shifted so as to clear a predetermined threshold; on the other hand, when an image has content that one does not want to extract the image as a scene change image as much as possible, even if an actual image-to-image change amount is large, the image-to-image change amount is shifted not to clear a predetermined threshold.

As a feature at the time of calculating an image-to-image change amount, the correlation between images, the SSD (sum of squared differences in pixel), the SAD (sum of absolute differences in pixel), and the like that have been commonly known can be used. Furthermore, a method of dividing an image into regions and performing a similar feature calculation on each region can be used; alternatively, points selected in a regular manner or at regular intervals and a highly-characterized local feature point are calculated, and an amount of motion or an optical flow of each point is obtained, and its magnitude can be used as an image change amount; if a value can be defined as a feature amount, the value can be used as a value for deriving a feature change amount in the present invention.

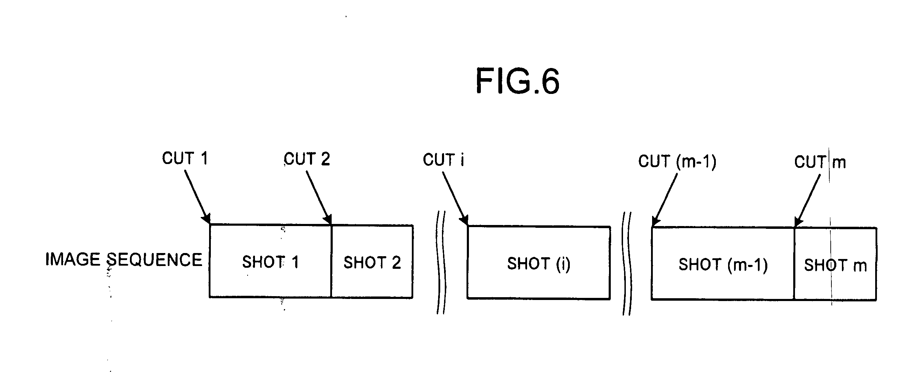

A continuous image sequence processed in the scene-change detection process according to the present first embodiment is, as shown in FIG. 6, that detected scene change images are cut and divided into a plurality of shots, and when the scene change images are actually displayed on the display unit 4, the scene change images are sequentially displayed from the first shot of the scene change image (cuts 1, 2, ..., i, ..., n-1, 1). In this display, an image having a small image-to-image change amount is not displayed. In other words, an image having a high degree of similarity is omitted from the display, so it is possible to display images efficiently. At this time, according to the present embodiment, as an image-to-image change amount, as described above, a statistic (an aggregate image change amount) is calculated, and the calculated value is used as an image-to-image change amount, and thus it is possible to detect a more effective scene change image reflecting image content than that is obtained by the conventional method.

In the above explanation, a case of calculating a change in feature between adjacent images in time-series order is explained; however, it is not particularly limited to a process between two adjacent images, and it can be configured that a feature among two or more images is calculated, a value corresponding to a feature change amount is calculated by the statistical operation of a combination of them, and then detection of the set number of images based on the ordering according to the value can be performed.

Furthermore, in a continuous image sequence, similar images may be continued; in such a case, a result of a process performed on an image can be applied to a plurality of continuous images. Namely, in the case of the present first embodiment, a feature region is extracted from an image, and a condition of image-to-image change detection is set on the basis of a feature amount of the feature region, so the same condition of image-to-image change detection is set with respect to a plurality of continuous images. Consequently, it is not necessary to perform a process for extraction/recognition of the feature region with respect to all images, and a processing time can be shortened.

As a technique for determining the number of images to which the above method is applied, simply, the predetermined number of images is just decided in advance, or can be adaptively decided by judging from comparison of an image-to-image similarity with a predetermined threshold.

Moreover, as a feature amount determining a level of importance of a feature region, any of gradation information, brightness information, position information, and size information of the feature region can be used.
For example, color gradation information or brightness information is useful information for determining a level of importance of the feature region, so when specific color or brightness information is recognized by using such information, a scene change image can be detected with a high degree of accuracy by setting a level of importance.

Furthermore, using a feature amount based on a position of a feature region as a feature amount of a region is also useful for setting a condition of detection depending on the feature amount of the feature region. Namely, which position within a screen (an image) a feature region is caught on has an association with a level of importance of the feature region, so a condition of detection is set in consideration of a level of importance associated with a position of the feature region, such as a way that if the feature region is caught on near the center of the screen (the image), a level of importance of the feature region is set high; if the feature region is caught on the corner of the screen (the image), a level of importance of the feature region is set low, whereby a scene change image associated with a composition of taken images can be detected effectively.

Moreover, from the viewpoint of the size of the feature region, setting of a level of importance is possible, and an effective scene change image can be detected by setting a level of importance based on the size.

### Second embodiment

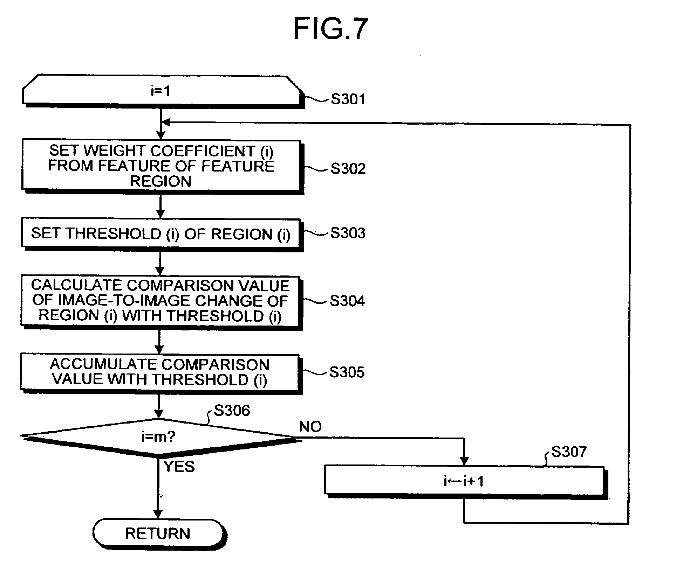

A second embodiment of the present invention is explained with reference to FIG. 7. FIG. 7 is a schematic flowchart illustrating a processing example according to the present second embodiment as an alternative to the processing example in FIG. 3. In the first embodiment, an image-to-image change amount weighted region by region of a target image is calculated and accumulated, and a change among a plurality of images is detected by using a statistic on the weighted image-to-image change amount; in the present second embodiment, by using a threshold weighted region by region of a target image, a difference between the threshold and an image-to-image change is calculated and accumulated, and a change among a plurality of images is detected by using a statistic on the difference. Namely, in the first embodiment, an image change amount is varied by weighting on a region-by-region basis; in the present second embodiment, a threshold is varied by b weighting on a region-by-region basis.

Also in FIG. 7, the number of regions into which each image is divided on the basis of extraction of a feature region is denoted by m, and a variable identifying each region is denoted by i. First, the variable i is set at 1 (Step S301). Then, the aggregate-change-amount calculating unit 232 sets a weight coefficient (i) of a region (i) on the basis of a feature amount of an extracted feature region of a corresponding image (Step S302), and sets a threshold (i) with respect to the region (i) in accordance with the set weight coefficient of the region (i) (Step S303). Namely, with respect to the region (i), the threshold (i) is weighted so that an image-to-image change of the region (i) is relatively weighted on the basis of the feature amount of the feature region, thereby setting a condition of image-to-image change detection. At this time, with respect to an initial threshold set in advance, it is appropriate that the threshold is set lower with increasing the weight coefficient so as to make it easy to detect, and the threshold is set higher with decreasing the weight coefficient so as to make it hard to detect. Then, the image-change detecting unit 231 calculates a difference between an image change of the region (i) with respect to an image to be compared and the set weighted threshold (i) as a comparison value (Step S304). Furthermore, the aggregate-change-amount calculating unit 232 accumulates the calculated comparison value with the threshold (i) thereby aggregating as a statistic (Step S305). Such processes are repeated in the same manner by incrementing the variable i by +1 until the variable i reaches m, the number of all the regions (Steps S306 and S307).

Also in the case of the present second embodiment, since a threshold is variably set by weighting on a region-by-region basis, a calculated statistic is a value taking a distribution of a level of importance of each region in a whole area of a target image into consideration, and a scene change image sequence is generated by detecting a scene change image by using the statistic calculated on an image-by-image basis as above, and thus it is possible to generate an appropriate scene change image sequence taking a feature amount of a feature region into consideration.

### Example

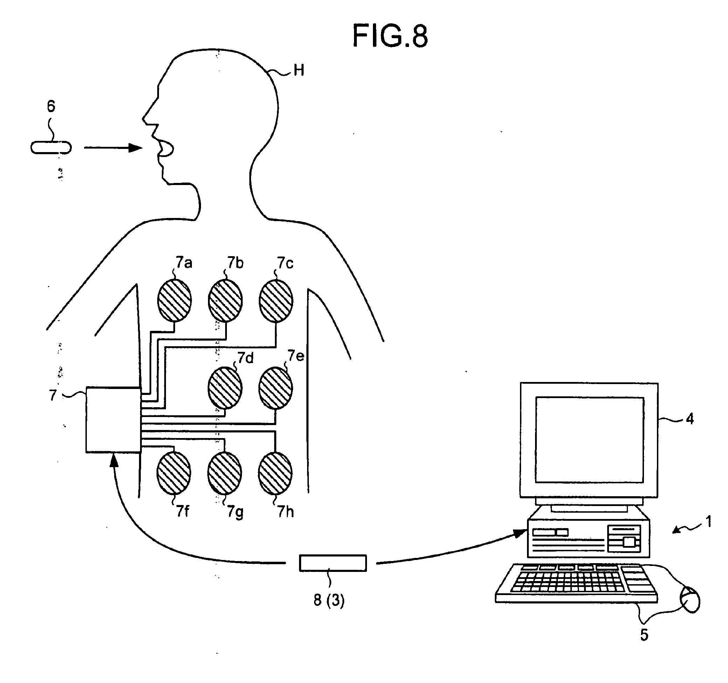

An example of the scene-change detection device according to the present invention is explained with reference to FIG. 8. The present example is that the scene-change detection device 1 according to the above first or second embodiment is used in a capsule endoscope system. FIG. 8 is a configuration diagram schematically illustrating a capsule endoscope system including the scene-change detection device 1 according to the present example as a workstation. The capsule endoscope system includes a capsule endoscope 6 which is introduced into a body cavity of a subject H and takes an image of inside the body cavity, a receiving device 7 which receives a radio signal transmitted from the capsule endoscope 6 and accumulates image information included in the received radio signal, and a portable storage unit 8, such as a memory card, which can be removably attached to the receiving device 7 and the scene-change detection device 1. The storage unit 8 corresponds to the storage unit 3 shown in FIG. 1.

The capsule endoscope 6 has an imaging function of taking an image of inside the body cavity of the subject H and a radio communication function of transmitting a radio signal including the taken image of inside the body cavity to the outside. More specifically, the capsule endoscope 6 takes images of inside the body cavity of the subject H at predetermined intervals (about 2 Hz), for example, about every 0.5 second while moving ahead inside the body cavity of the subject H, and transmits the taken images of inside the body cavity to the receiving device 7 through predetermined radio waves.

A plurality of receiving antennas 7a to 7h for receiving a radio signal transmitted from the capsule endoscope 6 are connected to the receiving device 7. The receiving antennas 7a to 7h are, for example, loop antennas, and arranged on the body surface to be distributed at positions corresponding to a pathway through which the capsule endoscope 6 passes. At least one such receiving antenna has to be arranged with respect to the subject H, and the number of receiving antennas arranged is not limited to eight as illustrated in the drawing.

The receiving device 7 receives a radio signal transmitted from the capsule endoscope 6 via any of the receiving antennas 7a to 7h, and acquires image information of an image of inside the body cavity of the subject H from the received radio signal. The image information acquired by the receiving device 7 is stored in the storage unit 8 attached to the receiving device 7. The storage unit 8 storing therein the image information of the image of inside the body cavity of the subject His attached to the scene-change detection device 1 to serve for a scene-change detection process in the control unit 2.

An object of such a capsule endoscope system is achieved in such a manner that by using the scene-change detection device 1 having the configuration as explained in the above first or second embodiment, a site of lesion or a site of bleeding is extracted/recognized as a feature region from an image in a sequence of images of inside the body cavity, or a target organ or mucous membrane is extracted/recognized as a feature region, and a condition of image-to-image change detection on a region-by-region basis is set to be varied on the basis of a feature amount of the feature region.

Namely, in a case of handling images of inside the body cavity taken by the capsule endoscope 6, a site of lesion, a site of bleeding, a mucous membrane, various valves, or the like on an image corresponds to an important feature region of the image, so a sufficient number of such images need to be preserved in a scene change image sequence (a summary image sequence). On the other hand, an image of contents suspended in the digestive tract, bubbles, outside of the body before the capsule endoscope 6 is put into the mouth, or the like is low in level of importance even if it includes a feature region, so if a lot of such images are preserved in a scene change image sequence (a summary image sequence), the scene change image sequence (the summary image sequence) is poor quality. Based on such circumstances, as for an image of inside the body cavity, extraction/recognition of a feature region as described above can be made from color information or brightness information of the image although it is a rough extraction/recognition. Consequently, in an image including a feature region of a high level of importance, a condition of detection is set so that an image-to-image change amount of the whole image is relatively increased; on the other hand, in an image including a feature region of a low level of importance, a condition of detection is set so that an image-to-image change amount of the whole image is relatively decreased.

This makes it possible to detect a scene change image depending on a level of importance specific to an image of inside the body cavity taken by the capsule endoscope 6, and thus it is possible to support an effective diagnosis.

### INDUSTRIAL APPLICABILITY

As described above, the scene-change detection device and the scene-change detection program according to the present invention are useful in detecting a scene change image at the position where a scene is changed in a sequence of continuously-taken images or a sequence of frame images of a moving image, and particularly suitable for detecting a scene change image in accordance with a feature of an image from a sequence of continuous images of inside a body cavity taken by a capsule endoscope.

## Claims

1. A scene-change detection device (1) configured for detecting a scene change from a continuous image sequence on the basis of an image-to-image change amount among a plurality of images, the scene-change detection device (1) comprising:
a feature-region extracting unit (22) that is configured to extract a feature region from a given image in the image sequence;
**characterized by** comprising a detecting unit (23) that is configured to:
set a condition of detecting an image as a scene-change image on the basis of a feature amount of the feature region extracted,
wherein the detecting unit (23) comprises an aggregate-change-amount calculating unit (232) configured to calculate a statistic of image-to-image change amounts of the entire area of the given image by¹ one of:
weighting image-to-image change amounts for a plurality of regions including the extracted feature region², and
weighting thresholds for the plurality of regions and determining differences between the weighted thresholds and the image-to-image change amounts for the plurality of regions³; and
detect a scene change among a plurality of images according to the calculated statistic,
wherein each of the image-to-image change amounts is one of a correlation, a magnitude of motion, or a magnitude of optical flow between the plurality of images.

2. The scene-change detection device (1) according to claim 1, wherein the plurality of regions in a whole area of the image includes the feature region extracted, a region other than the feature region, and a common region and a mismatched region between the image and an image to be compared that are generated in accordance with movement of the feature region with respect to the image to be compared.

3. The scene-change detection device (1) according to claim 1 or 2, wherein the detecting unit (23) sets the same condition of image change detection with respect
¹ [19], co. 5, lines 3 to 12; [42], col. 12, lines 14 to 16
² [19], col. 5, lines 7 to 12; [24], col. 7, lines 7 to 9; [28], first sentence.
³ [42], col. 12, lines 1 to 3 and 14 to 16.
to a plurality of continuous images in the continuous image sequence.

4. The scene-change detection device (1) according to any one of claims 1 to 3,
wherein at least any one of gradation information, brightness information, position information, and size information of the feature region is used as a feature amount of the feature region.

5. The scene-change detection device (1) according to any one of claims 1 to 4,
wherein the continuous image sequence is a sequence of images of inside a body cavity taken by a capsule endoscope (6) introduced into the body cavity of a subject (H).

6. The scene-change detection device (1) according to claim 5, wherein the feature region extracted by the feature-region extracting unit (22) includes a region of a site of lesion, a site of bleeding, or a mucous membrane included in the images of inside the body cavity.

7. A scene-change detection program for causing a computer to execute the step of detecting a scene change from a continuous image sequence on the basis of an image-to-image change amount among a plurality of images, the scene-change detection program causing the computer to execute the steps of:
extracting a feature region from an image in the image sequence; and
setting a condition of detecting a scene change from among the plurality of images on the basis of a feature amount of the feature region extracted;
**characterized by** further comprising the steps of:
calculating a statistic of an image-to-image change amount of the entire area of the given image by one of:
weighting image-to-image change amounts amounts for each of a plurality of regions including the extracted feature region⁴, and
weighting thresholds for each of the plurality of regions and
determining differences between the weighted thresholds and the image-to-image change amounts for each of the plurality of regions⁵; and
detecting a scene change among the plurality of images according to the calculated statistic and the differences between the weighted thresholds,
wherein each of the image-to-image change amounts is one of a correlation, a magnitude of motion, or a magnitude of optical flow between the plurality of images.
⁴ [19], col. 5, lines 7 to 12; [24], col. 7, lines 7 to 9; [28], first sentence.
⁵ [42], col. 12, lines 1 to 3 and 14 to 16.

## Patentansprüche

1. Szenenwechsel-Ermittlungsvorrichtung (1), die dazu ausgebildet ist, einen Szenenwechsel aus einer fortlaufenden Bildfolge auf der Grundlage eines Bild-zu-Bild-Wechselumfangs unter einer Vielzahl von Bildern zu ermitteln, wobei die Szenenwechsel-Ermittlungsvorrichtung (1) aufweist:
eine Merkmalsregion-Extraktionseinheit (22), die dazu ausgebildet ist, eine Merkmalsregion aus einem gegebenen Bild in der Bildfolge zu extrahieren;
**dadurch gekennzeichnet, dass** sie eine Ermittlungseinheit (23) aufweist, die zu Folgendem ausgebildet ist:
Einstellen einer Bedingung zum Ermitteln eines Bildes als ein Szenenwechsel-Bild auf der Grundlage eines Merkmalsumfangs der extrahierten Merkmalsregion,
wobei die Ermittlungseinheit (23) eine Gesamtwechselumfang-Berechnungseinheit (232) aufweist, die eine Statistik der Bild-zu-Bild-Wechselumfänge des gesamten Bereichs des gegebenen Bildes berechnet durch eines von:
Gewichten von Bild-zu-Bild-Wechselumfängen für eine Vielzahl von Regionen, einschließlich der extrahierten Merkmalsregion, und
Gewichten von Schwellenwerten für die Vielzahl von Regionen und Bestimmen von Unterschieden zwischen den gewichteten Schwellenwerten und den Bild-zu-Bild-Wechselumfängen für die Vielzahl von Regionen; und
Ermitteln eines Szenenwechsels unter einer Vielzahl von Bildern entsprechend der berechneten Statistik,
wobei jeder der Bild-zu-Bild-Wechselumfänge einer ist von einer Korrelation, einer Größe der Bewegung oder einer Größe des optischen Flusses zwischen der Vielzahl von Bildern.

2. Szenenwechsel-Ermittlungsvorrichtung (1) nach Anspruch 1, wobei die Vielzahl von Regionen in einem ganzen Bereich des Bildes die extrahierte Merkmalsregion, eine andere Region als die Merkmalsregion sowie eine gemeinsame Region und eine nicht zusammenpassende Region zwischen dem Bild und einem zu vergleichenden Bild, die gemäß Bewegung der Merkmalsregion bezüglich des zu vergleichenden Bildes erzeugt werden, einschließt.

3. Szenenwechsel-Ermittlungsvorrichtung (1) nach Anspruch 1 oder 2, wobei die Ermittlungseinheit (23) dieselbe Bedingung der Bildwechselermittlung bezüglich einer Vielzahl von fortlaufenden Bildern in der fortlaufenden Bildfolge einstellt.

4. Szenenwechsel-Ermittlungsvorrichtung (1) nach einem beliebigen der Ansprüche 1 bis 3, wobei mindestens eine von Gradationsinformationen, Helligkeitsinformationen, Positionsinformationen und Größeninformationen der Merkmalsregion als ein Merkmalsumfang der Merkmalsregion verwendet wird.

5. Szenenwechsel-Ermittlungsvorrichtung (1) nach einem beliebigen der Ansprüche 1 bis 4, wobei die fortlaufende Bildfolge eine Folge von Bildern vom Inneren eines Körperhohlraums ist, die durch ein Kapselendoskop (6) aufgenommen werden, das in den Körperhohlraum eines Patienten (H) eingeführt ist.

6. Szenenwechsel-Ermittlungsvorrichtung (1) nach Anspruch 5, wobei die durch die Merkmalsregion-Extraktionseinheit (22) extrahierte Merkmalsregion eine Region von einer Läsionsstelle, einer Blutungsstelle oder einer Schleimhaut aufweist, die in den Bildern vom Innern des Körperhohlraums enthalten ist.

7. Szenenwechsel-Ermittlungsprogramm, das einen Computer veranlasst, den Schritt des Ermittelns eines Szenenwechsels aus einer fortlaufenden Bildfolge auf der Grundlage eines Bild-zu-Bild-Wechselumfangs unter einer Vielzahl von Bildern auszuführen, wobei das Szenenwechsel-Ermittlungsprogramm den Computer veranlasst, die folgenden Schritte auszuführen:
Extrahieren einer Merkmalsregion aus einem Bild in der Bildfolge; und
Einstellen einer Bedingung zum Ermitteln eines Szenenwechsels unter der Vielzahl von Bildern auf der Grundlage eines Merkmalsumfangs der extrahierten Merkmalsregion;
**dadurch gekennzeichnet, dass** es des Weiteren folgende Schritte aufweist:
Berechnen einer Statistik eines Bild-zu-Bild-Wechselumfangs des gesamten Bereichs des gegebenen Bildes durch eines von:
Gewichten von Bild-zu-Bild-Wechselumfängen für jede einer Vielzahl von Regionen, einschließlich der extrahierten Merkmalsregion, und
Gewichten von Schwellenwerten für jede der Vielzahl von Regionen und Bestimmen von Unterschieden zwischen den gewichteten Schwellenwerten und den Bild-zu-Bild-Wechselumfängen für jede der Vielzahl von Regionen;
und
Ermitteln eines Szenenwechsels unter der Vielzahl von Bildern entsprechend der berechneten Statistik und den Unterschieden zwischen den gewichteten Schwellenwerten,
wobei jeder der Bild-zu-Bild-Wechselumfänge einer ist von einer Korrelation, einer Größe der Bewegung oder einer Größe des optischen Flusses zwischen der Vielzahl von Bildern.

## Revendications

1. Dispositif de détection de changement de scène (1) configuré pour détecter un changement de scène à partir d'une séquence d'images continues sur la base d'une quantité de changements image par image parmi une pluralité d'images, le dispositif de détection de changement de scène (1) comprenant :
une unité d'extraction (22) de région de caractéristiques qui est configurée pour extraire une région de caractéristiques d'une image donnée dans la séquence d'images ;
**caractérisé en ce que** comprenant une unité de détection (23) qui est configurée pour :
fixer une condition de détection d'une image comme une image de changement de scène sur la base d'une quantité de caractéristiques de la région de caractéristiques extraite,
dans lequel l'unité de détection (23) comprend une unité de calcul (232) de quantité totale de changements configurée pour calculer une statistique de quantités de changements image par image de la totalité de la superficie de l'image donnée par une parmi :
la pondération de quantités de changements image par image pour une pluralité de régions incluant la région de caractéristiques extraite, et
la pondération de seuils pour la pluralité de régions et la détermination de différences entre les seuils pondérés et les quantités de changements image par image pour la pluralité de régions ; et
détecter un changement de scène parmi une pluralité d'images en fonction de la statistique calculée,
dans lequel chacune des quantités de changements image par image est une parmi une corrélation, une grandeur de mouvement, ou une grandeur de flux optique entre la pluralité d'images.

2. Dispositif de détection de changement de scène (1) selon la revendication 1, dans lequel la pluralité de régions dans une totalité de la superficie de l'image inclut la région de caractéristiques extraite, une région autre que la région de caractéristiques, et une région commune et une région sans correspondance entre l'image et une image devant être comparée qui sont générées conformément à un mouvement de la région de caractéristiques par rapport à l'image devant être comparée.

3. Dispositif de détection de changement de scène (1) selon la revendication 1 ou 2, dans lequel l'unité de détection (23) fixe la même condition de détection de changement d'image par rapport à une pluralité d'images continues dans la séquence d'images continues.

4. Dispositif de détection de changement de scène (1) selon l'une quelconque des revendications 1 à 3, dans lequel au moins une quelconque parmi une information de gradation, une information de luminosité, une information de position, et une information de taille de la région de caractéristiques est utilisée comme une quantité de caractéristiques de la région de caractéristiques.

5. Dispositif de détection de changement de scène (1) selon l'une quelconque des revendications 1 à 4, dans lequel la séquence d'images continues est une séquence d'images de l'intérieur d'une cavité corporelle prise par un endoscope de type capsule (6) introduit à l'intérieur de la cavité corporelle d'un sujet (H).

6. Dispositif de détection de changement de scène (1) selon la revendication 5, dans lequel la région de caractéristiques extraite par l'unité d'extraction (22) de région de caractéristiques inclut une région d'un site de lésion, d'un site de saignement, ou d'une membrane muqueuse incluse dans les images de l'intérieur de la cavité corporelle.

7. Programme de détection de changement de scène pour faire en sorte qu'un ordinateur exécute l'étape de détection d'un changement de scène à partir d'une séquence d'images continues sur la base d'une quantité de changements image par image parmi une pluralité d'images, le programme de détection de changement de scène faisant en sorte que l'ordinateur exécute les étapes de :
extraction d'une région de caractéristiques d'une image dans la séquence d'images ; et
fixation d'une condition de détection d'un changement de scène parmi la pluralité d'images sur la base d'une quantité de caractéristiques de la région de caractéristiques extraite ;
**caractérisé en ce que** comprenant en outre les étapes de :
calcul d'une statistique d'une quantité de changements image par image de la totalité de la superficie de l'image donnée par une parmi :
la pondération de quantités de changements image par image pour chacune d'une pluralité de régions incluant la région de caractéristiques extraite, et
la pondération de seuils pour chacune de la pluralité de régions et la détermination de différences entre les seuils pondérés et les quantités de changements image par image pour chacune de la pluralité de régions ; et
détection d'un changement de scène parmi la pluralité d'images en fonction de la statistique calculée et des différences entre les seuils pondérés,
dans lequel chacune des quantités de changements image par image est une parmi une corrélation, une grandeur de mouvement, ou une grandeur de flux optique entre la pluralité d'images.
